# EUROPEAN PATENT SPECIFICATION

(11) **EP 3 923 889 B1**
(45) Date of publication and mention of the grant of the patent: **03.04.2024**
(21) Application number: 19914736.4
(22) Date of filing: 15.02.2019
(51) Int. Cl.: A61F 13/511, A61F 13/47, A61F 13/51

(54) **ABSORBENT ARTICLE WITH EMBOSSED SURFACE LAYER**
SAUGFÄHIGER ARTIKEL MIT GEPRÄGTER OBERFLÄCHENSCHICHT
ARTICLE ABSORBANT POURVU D'UNE COUCHE DE SURFACE GAUFRÉE

(43) Date of publication of application: 22.12.2021
(73) Proprietor: Essity Hygiene and Health Aktiebolag, 405 03 Göteborg (SE)
(72) Inventor: BLOMSTRÖM, Philip, 405 03 GÖTEBORG (SE); ABBAS, Shabira, 405 03 GÖTEBORG (SE)
(74) Representative: Hoffmann Eitle
(86) International application number: PCT/SE2019/050131
(87) International publication number: WO 2020/167172

(56) References cited:
- WO-A1-2010/074208
- WO-A1-2010/074208
- WO-A1-2011/155284
- WO-A1-2011/155284
- JP-A- 2017 029 494
- US-A1- 2003 187 417
- US-A1- 2003 187 417
- US-A1- 2006 142 724
- US-A1- 2006 142 724
- US-A1- 2012 238 984
- US-A1- 2014 066 873
- US-A1- 2014 343 525
- US-A1- 2015 164 709
- US-A1- 2015 182 387
- US-A1- 2016 220 421

## Description

### TECHNICAL FIELD

The present invention relates to an absorbent article. The present invention in particular relates to an absorbent article having a soft surface layer with an improved embossing pattern.

### BACKGROUND OF THE INVENTION

Disposable absorbent articles, such as pantiliners, sanitary napkins, adult incontinence devices and diapers, of the kind to which this disclosure relates are worn against the skin and are used to absorb bodily fluids. The absorbent articles include a topsheet and a backsheet layer, and may also conventionally include an absorbent core therebetween.

All uses of products which are applied in direct contact with the skin sometimes experience skin problems. Skin problems can be caused by forces arising from physical/mechanical interaction between the absorbent product and the user's skin. Thus, for example chafing is caused due to extra friction between the absorbent product and the skin of the user. The mechanical friction between the material and the skin of the user is different in the presence of liquid/moisture than when no liquid/moisture is present.

Absorbent articles may have printed or embossed patterns imposed upon their surfaces. For example, sanitary protection articles, such as, napkins, pantiliners, and incontinence devices, typically have a liquid permeable surface layer provided with a pattern of depressed areas embossed into the surface in such configurations as flowers or other feminine designs. Other patterns may take the form of various geometric shapes, such as, circles, diamonds, squares, curves, or other stylized figures, such as, stars, spots, or the like.

While articles are embossed mainly for decorative purposes, embossing may communicate or provide a function to the user of such an article. For example, it is known that embossing functions in impeding or directing fluid flow. Embossing is also done to provide a visual cue to show differences in the material surface. To provide the desired aesthetical effect, embossing patterns may usually be imprinted relatively deep; e.g., the depressed areas may be permanently depressed to a degree, which represents a major portion of the thickness of the product.

WO 2010/074208 A1 discloses a non-woven fabric which comprises composite fibers each comprising a first component and a second component having different melting points from each other, and which has, formed therein, many thermally fused points produced by thermally fusing the intersection points of the composite fibers. When the state of the non-woven fabric is changed from dried state to wet state, the percentage of change in thickness of the non-woven fabric is 15% or more. In a non-woven fabric which is an embodiment of the non-woven fabric, the composite fibers is fibers each comprising a polypropylene resin or a polyethylene terephthalate resin as the first component and a resin having a lower melting point from that of the first component as the second component and has a Young's modulus of 0.2 to 1.0 GPa.

WO 2011/155284 A1 discloses a sanitary napkin comprising a topsheet, a backsheet, an absorbent, and a second sheet, the absorbent and the second sheet being disposed between the topsheet and backsheet. The topsheet and the second sheet have been partly bonded to each other. The topsheet has regions separated by linear grooves formed by embossing, and is a sheet that satisfies a given requirement in an examination for pore diameter distribution with a mercury porosimeter. The second sheet is constituted of synthetic fibers and is a sheet having a thickness and a liquid-holding capacity that satisfy respective given requirements.

US 2014/343525 A1 discloses an absorbent personal care article, such as a sanitary napkin or incontinence pad having a longitudinal centerline and a transverse centerline and including a pair of opposed first and second wings extending along the longitudinal sides of the article. The article further includes first and second protective strips attached to the sides of the article such that when the wings are folded under the article and around the wearer's undergarment, the first and second protective strips extend laterally outboard of the article and the edges of the wearer's undergarment to provide additional protection against leakage of body fluids deposited onto the absorbent personal care article.

US 2006/142724 A1 discloses a sanitary napkin that includes: a napkin body having a liquid-absorbent layer for absorbing and retaining liquid; and a projection projecting from a body surface of the napkin body. The projection has an apex and a pair of side wall portions formed of a liquid-permeable material. The apex is spaced apart from the body surface of the napkin body and extends along a longitudinal centerline of the napkin body. The side wall portions connect at the apex and diverge toward the body surface of the napkin body. The apex and the side wall portions are different in stiffness. The side wall portions have a space therebetween and are permitted to deform and approach each other.

US 2003/187417 A1 discloses an absorbent article including a liquid-permeable top layer, a backsheet, and an absorbent layer disposed between the top layer and the backsheet. Fibers constituting the absorbent layer are bonded together. The absorbent layer includes hydrophilic fibers. The top layer and the absorbent layer are embossed together to have recesses in which fibers constituting the absorbent layer are bonded to the top layer.

It is an object of the present invention to provide an absorbent article having a distinct embossing pattern as well as having improved skin benefits.

### SUMMARY OF THE INVENTION

One or more of the above objects may be achieved with an absorbent article in accordance with claim 1. Further embodiments are set out in the dependent claims, in the following description and in the drawings.

The absorbent article as disclosed herein has longitudinal side edges extending in a longitudinal direction and transverse front and rear end edges extending in a transverse direction. The absorbent article comprises a fluid permeable surface layer and a backsheet. The fluid permeable surface layer is an embossed surface layer comprising an embossing pattern covering from 3% to 20% of the total surface area of a wearer-facing portion of said surface layer. The surface layer is an air-through-bonded fibrous nonwoven surface layer comprising synthetic fibers and has a basis weight of from 14 to 30 g/m².

The term "absorbent articles" refers to products that are placed against the skin of the wearer to absorb and contain body exudates, like urine, faeces and menstrual fluid. The disclosure mainly refers to disposable absorbent articles, which means articles that are not intended to be laundered or otherwise restored or reused as an absorbent article. Examples of disposable absorbent articles include feminine hygiene products such as sanitary napkins and panty liners, incontinence pads and diapers and the like.

The air-through-bonded fibrous nonwoven surface layer having a basis weight of from 14 to 30 g/m² has been found to enhance the optical appearance of an embossing pattern by increasing the distinctness of the embossing pattern. One reason may be that such an air-through bonded fibrous nonwoven has relatively low number of bonding points between the fibers within the material which enhances the visibility of the embossing pattern when provided on the material.

The synthetic fibers may be bicomponent fibers. The bicomponent fibers may have a sheath of polyethylene or polyprophylene. The core may be of polyester. To use synthetic fibers in the air-through-bonded fibrous nonwoven wherein at least the sheath of the fiber is of polyethylene has also been seen to render the embossing pattern more distinct which may result from breaking of the fiber structure during embossing.

To use synthetic fibers in the air-through-bonded fibrous nonwoven wherein the sheath of the fiber is of polyester (PET) has also been seen to render the surface material more resilient.

Air-through-bonded fibrous nonwoven material comprising sheath-core bicomponent fibers, and particularly wherein the core is a polyester core and the sheath is a polyethylene sheath, has been seen to provide improved and distinct embossing patterns which may result from breaking of the bicomponent fiber structure during embossing and the polyester core is beneficial for enhancing the resiliency of the nonwoven structure.

The fluid permeable surface layer is an embossed surface layer comprising an embossing pattern covering from 3% to 20%, or 5% to 16%, of the total surface area of a wearer-facing portion of said surface layer. This has been found to provide a soft and compliant surface sheet with a good visibility of the embossed elements. If a too large extent of the surface area of the surface layer is provided with embossing pattern, the surface layer becomes too stiff. The fluid permeable surface layer may have an embossing free area in the crotch portion of the article corresponding to the liquid inlet area so as to maintain a relatively low density (=high bulk) in the liquid inlet area which is beneficial for the liquid inlet rate.

By "wearer-facing portion" of the surface layer means the portion of the surface layer facing the wearer during use of the absorbent article. The absorbent article may for example comprise wings or flaps provided with attachment means, such as adhesive. The surface layer may extend over the wings of the absorbent article, however the "wearer-facing portion" does not include wings or flaps as these are not intended to face the wearer during use. To determine the "wearer-facing portion" of the surface layer for an absorbent article comprising wings or flaps, a straight line is drawn between a starting point and an end point of the respective wing along the respective longitudinal side of the absorbent article, i.e. the start and end point being where the contour of the absorbent article curve outwardly to form the respective wings.

The embossing pattern may comprise embossed continuous and/or discontinuous lines having a minimum width of 0.3 mm or 0.6 mm or 0.9 mm. The maximum width may be 5.0 mm, or 3.0 mm. The discontinuous lines may comprise or consist of embossed dots arranged in a discontinuous line, such as a straight or a curved line and/or embossed pattern of different kind. The embossed dots may have a minimum diameter of 0.3 mm, or 0.6 mm or 0.9. The maximum diameter may be 5.0 mm, or 3.0 mm.

The embossing pattern may have a minimum depth of 0.3 mm, 0.4 mm or 0.5 mm. The maximum depth may be 3 mm.

The bi-component fibers in the embossing pattern may be permanently deformed but not consolidated.

The surface layer according to the present invention has a density of from 20 to 90 kg/m³, 20 to 60 kg/m³, 20 to 40,15 to 40 kg/m³ or 20 to 30 kg/m³. The air-through-bonded surface layer has a relatively low density, and this means that there is a relatively high amount of void space between the fibres. The density can be calculated by dividing the basis weight of the surface layer by its thickness measured at a pressure of 0.5 kPa
The fibers of said air-through-bonded nonwoven surface layer may have a coarseness of from 1.8 to 10 dtex, or 2 to 7 dtex or 3.5 to 7 dtex.

The surface layer may be free from lotions and/or lubricating agents. As the surface layer in it-self has been found to provide the surface layer with surprisingly low friction values both under dry and wet conditions, lotions and lubricant agents may not be needed to decrease the friction between the nonwoven and the user's skin.

The surface material may be hydrophilic. A hydrophilic material may be obtained by adding a surfactant.

The absorbent article may comprise an intermediate layer, such as an intermediate fibrous layer, located between the surface layer and the backsheet and in direct contact with the surface layer. The intermediate layer may be a nonwoven layer such as airlaid or high-loft nonwoven materials, such as for example air-through-bonded nonwoven or hydroentangled nonwoven material.

The intermediate layer may extend under from 70% to 100% of the wearer-facing portion of the surface layer.

The surface layer and the intermediate layer may be adhesively attached to each other. This may increase the integrity of the surface layer. The fact that the surface layer has a relatively low density with relatively low number of bonding points between the fibers gives a structure with lower integrity. However, when combining the air-through-bonded fibrous nonwoven surface layer with an intermediate layer having a lower elongation than the air-through-bonded fibrous nonwoven, the air-through-bonded fibrous nonwoven surface layer integrity is increased. Both during use of the absorbent product as well as during manufacturing it is advantageous that a structure(s) has a sufficient integrity.

It is also possible to attach the liquid surface material with the intermediate layer by thermo- and or mechanical welding, for example by ultrasonic welding. By this, the lamination and embossing may be done in the same step.

According to one embodiment, only the fluid permeable surface layer of the sanitary is embossed and not the intermediate layer. However, according to another embodiment the embossing is done also through the intermediate layer, so that also the intermediate layer is embossed. Furthermore, it is also possible to emboss through the surface layer, the intermediate layer and also through a further layer such as for example an absorbent core which may be arranged between the intermediate layer and a backsheet. If the liquid surface material is laminated together with the intermediate layer through thermo- and or mechanical welding, the lamination and embossing may be done in the same step. One embossment may penetrate both the fluid permeable surface layer and the intermediate layer, for example by ultrasonic welding.

The backsheet has a garment facing side and an adhesive may be arranged on said garment facing side.

The absorbent article may include an absorbent core arranged between the surface layer and the backsheet and between the intermediate layer and the backsheet if the absorbent article comprises an intermediate layer. The absorbent core may for example include pulp fibers or may comprise a mixture of superabsorbent particles and pulp fibers.

The backsheet may be a breathable or non-breathable plastic film. The backsheet may be a polyolefin plastic film. The backsheet may also be a laminate of a plastic film and a nonwoven material.

The absorbent article may be a sanitary napkin.

### BRIEF DESCRIPTION OF THE DRAWINGS

The present invention will be further explained hereinafter by means of non-limiting examples and with reference to the appended drawings wherein:
- Fig. 1: shows a top plan view of a sanitary napkin as disclosed herein and as seen from a topsheet side; and
- Fig. 2: shows an exploded perspective view the sanitary napkin of Fig. 1.
- Fig 3a: shows enlarged photographs of surface materials comprising discontinuous embossing dots;
- Fig 3b: shows enlarged photographs of surface materials comprising embossing continuous lines;
- Fig 4: shows friction measurement result on surface materials.

### DETAILED DESCRIPTION

The invention will be described more closely below by reference to an exemplary embodiment. The invention may however be embodied in many different forms and should not be construed as limited to the embodiments set forth in the drawings and the description thereto.

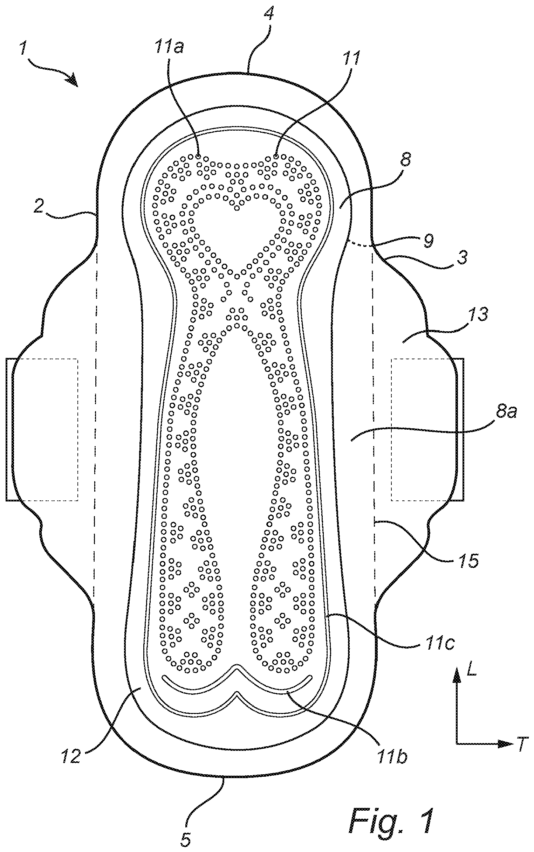

Figure 1 is a top plan view of a sanitary article 1 having longitudinal side edges 2,3 extending in a longitudinal direction L and transverse front and rear end edges 4,5 extending in a transverse direction T. The sanitary article 1 comprising a fluid permeable surface layer 8 and a backsheet 9. The surface layer 8 is an air-through-bonded fibrous nonwoven surface layer having a basis weight of from 14 to 30 g/m². The nonwoven layer comprises bicomponent fibers and may constitute of from 50% or more, such as 80% to 100% or from 95% or more, of bicomponent fibers. The bicomponent fibers may be sheath-core bicomponent fibers, for example the core is a polyester core and the sheath is a polyethylene sheath.

The fluid permeable surface layer 8 comprises an embossed pattern 11. The embossed pattern 11 comprises individual embossed elements 11a in the form of dots forming a pattern covering from 3 % to 20% of the wearer-facing portion of the surface layer 8. At a rear end 12 of the absorbent article 1, the surface layer 8 is provided with an embossed wing-shaped continuous line 11b and a continuous embossed line extends along a contour of the absorbent article 1 framing the embossed pattern 11.

The absorbent article 1 in fig. 1 is a sanitary napkin and is provided with a pair of lateral wings 13 extending outward from the transversely opposite side edges 2,3 of the napkin. The wings are provided with attachment means, such as with an adhesive, on their garment facing surface so that the wings 13 can be folded back under the undergarment and attached to the undergarment. In this way, the wings 13 serve to keep the napkin 1 properly positioned in the undergarment.

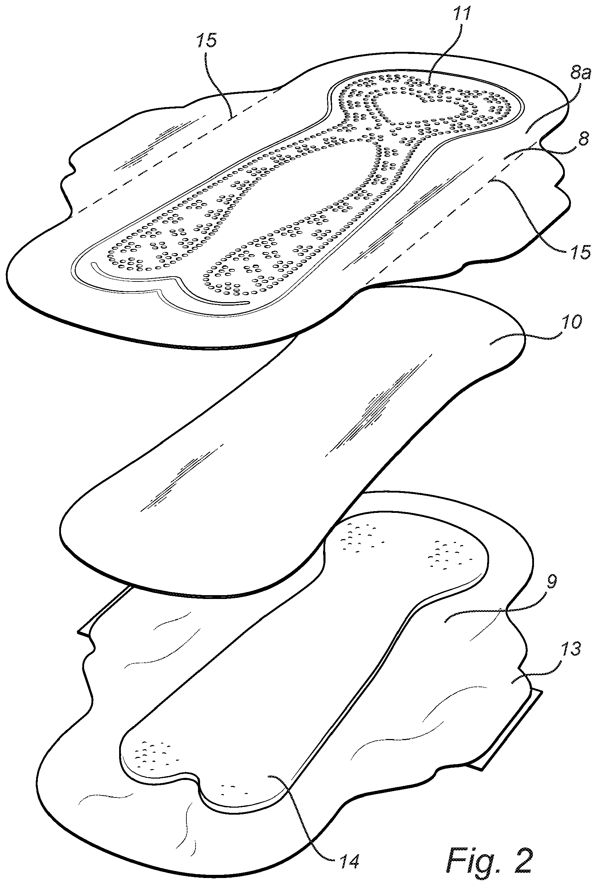

Fig. 2 is an exploded view of the sanitary napkin 1 shown in Fig. 2 and illustrates the sanitary napkin with the layer of the sanitary napkin 1 separated. The sanitary napkin 1 comprises a fluid permeable surface layer 8 and a backsheet 9. An intermediate fibrous layer 10 is located between the surface layer 8 and the backsheet 9 and in direct contact with the surface layer 8. In this figure the intermediate layer is provided under about 100% of the total surface area of a wearer-facing portion of the surface layer 8 and is adhesively attached to the surface layer 8. An absorbent core 14 is arranged between the intermediate layer 10 and the backsheet 9. Only the fluid permeable surface layer 8 of the sanitary napkin 1 in figure 2 is embossed and not the intermediate layer 10. However, also the intermediate layer may be embossed, and the embossing may be done in one step so that the embossments are penetrating both the fluid permeable surface layer 8 and the intermediate layer 10 in the same embossing step. It is also possible to laminate the liquid surface material 8 together with the intermediate layer 10 through thermo- and or mechanical welding, for example by ultrasonic welding. By this, the lamination and embossing are done in the same step.

The backsheet may be a breathable or non-breathable plastic film. The backsheet may be a laminate of a breathable or non-breathable plastic film and a nonwoven material.

The absorbent core may be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent structure. It is also common to have absorbent structures comprising layers of different material with different properties with respect to liquid acquisition capacity, liquid distribution capacity and storage capacity. This is well-known to the person skilled in the art and does therefore not have to be described in detail. The thin absorbent bodies, which are common in today's sanitary articles, often comprise a compressed mixed or layered structure of cellulosic fluff pulp and superabsorbent. The size and absorbent capacity of the absorbent structure may be varied to be suited for different uses such as sanitary articles, pantyliners, adult incontinence pads and diapers, baby diapers, pant diapers, etc.

The intermediate layer may be composed of for example airlaid nonwoven, high loft nonwoven such as for example air-through bonded nonwoven or hydroentangled nonwoven. An air laid nonwoven can be produced with fluff, wood pulp, and here the fluff fibres are dispersed into a fast-moving air stream and condensed onto a moving screen by means of pressure and vacuum. The web can be bonded with resin and/or thermal plastic resin dispersed within the pulp. The web can be thermobonded (by heat), latex bonded (with adhesive) or multibonded (a combination of thermo and latex bonding) or mechanically bonded (high compression and temperature, bonding by hydrogen). The grammage of the airlaid nonwoven can suitably be from 50 to 100 gsm.

High loft is a nonwoven material and may be substantially free from absorbing fibres and superabsorbent material. The high loft nonwoven material may comprise thermoplastic polymer fibres, and may be selected from but not limited to, polyesters, polyamides and polyolefins such as polyethylenes (PE) and polypropylenes (PP), and may be a mixture of any of these. The "high loft" refers to low density bulky fabrics, as compared to flat, paper-like fabrics. High loft webs are characterized by a relatively low density. This means that there is a relatively high amount of void space between the fibres. The intermediate high loft nonwoven layer may typically have a density below 200 kg/m³, in particular ranging from 15 kg/m³ to 150 kg/m³, in particular from 30 to 100 kg/m³. The average density can be calculated by dividing the basis weight of the high loft layer by its thickness measured at a pressure of 0.5 kPa. Normally the thickness of the intermediate layer of high loft material is more than about 0.5 mm, such as more than 1 mm or suitably 1.5 - 2.0 mm, and the solid content is low, usually less than 15 % by volume.

The raw material for the intermediate layer may be polyolefines, for example be polypropylene (PP), polyethylene (PE), or polyester (PET), polyamide (PA), cellulosic fibres or a combination of these. Thus, if a combination of different fibres is used, this can be a mixture of fibres from different polymers, although each fibre can also include different polymers (e.g. PP/PE bi-component fibres or PP/PE copolymers).

Where appropriate, the plastic backsheet film may comprising PE or PP, PET, PLA or amyl (or, for that matter, any other thermoplastic polymer), or a mixture or copolymers of the aforementioned polymers.

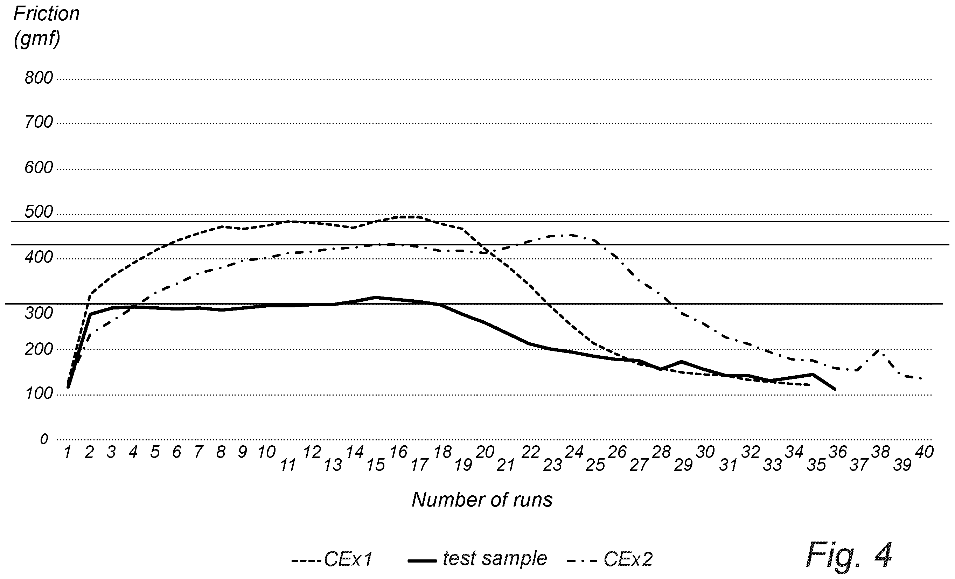

In Figure 4 shows the friction curves for test sample, CEx 1 and CEx2. In figure 4 is the number of runs on the x-axis and the friction force in gmf on the y-axis. A friction curve comprises a first slope having a positive coefficient illustrating increase in the friction values, a plateau, and a second slope having a negative coefficient illustrating decrease in friction values. At the plateau, the friction values are substantially constant over the extension of the plateau. Small variations at the plateau as well as along the slopes are possible between individual values, but with a positive coefficient is meant that all individual values in the first slope together creates a positive coefficient, as well as all individual values in the second slope together creates a negative coefficient, as well as all individual values in the plateau together creates a plateau. Lower friction values render the absorbent article more skin friendly and skin problems arising with the use of the absorbent article can be reduced. For some materials a clear peak can be seen in a curve of friction values before the second slope creating a negative coefficient. Such a peak is caused by clinging, which may occur when only a small amount of moisture is present. The result in Figure 4 shows that the test sample, the air-through-bonded nonwoven surface material, has a lower mean friction plateau value (gmf).

### Embossing measurement

The depth of the individual embossed elements in the form of dots have been measured by the method ISO25178 and also the depth of a continuous embossed line extending along a contour of the absorbent sanitary napkin framing the embossed pattern comprising the individual embossed elements have been measured by the method ISO25178.

Three different sanitary napkins were tested having different surface layers but otherwise constructed with the same underlying materials and compared in terms of diversity of embossment depth. The test material is an air-through-bonded nonwoven according to the present disclosure comprising bicomponent fibers of core-sheath type with a polyester core and a polyethylene sheath. The first Comparative Example is a spunbond nonwoven with polypropylene fibers and the second Comparative Example is spunbond nonwoven with polypropylene fibers. Table 1 below provides specifications of the materials tested.

**Table 1**

| | Material Type | Supplier | Material no | Basis weight (gsm) |
|---|---|---|---|---|
| Test sample | Air-though bonded nw | TWE | 255272 | 20 |
| CEx 1 | Spunbond nonwoven | Texbond | 2436701 | 18 |
| CEx 2 | Spunbond nonwoven | Union | 272119 | 18 |

In Table 2 shows the result of the mean individual depth of the individual embossed elements (µm) and the standard deviation of the depth (µm).

**Table 2**

| | Mean individual depth (µm) | Standard deviation (µm) |
|---|---|---|
| Test sample | 388 | 5.9 |
| CEx 1 | 446 | 26.0 |
| Cex 2 | 368 | 22.3 |

The result shows that the standard deviation of the depth value of the embossment element for the air-through-bonded nonwoven. The standard depth deviation for the Test sample was lower than the standard depth deviation for the comparative example 1 (Cex 1) and the comparative example 2 (Cex 2).

**Table 3 - The depth of a continuous embossed line, a "valley"**

| | Mean individual depth (µm) | Standard deviation (µm) | Ratio between Standard deviation and Mean individual depth |
|---|---|---|---|
| Test sample | 971 | 148 | 0.15 |
| CEx 1 | 597 | 204 | 0.34 |
| Cex 2 | 442 | 91 | 0.21 |

The mean individual depth of a continuous embossed line, a "valley", for the air-through-bonded nonwoven according to the Test sample, as seen in table 3, has a lower ratio between Standard deviation and Mean individual depth than the Comparative example 1 (Cex 1) and the Comparative example 2 (Cex 2).

So, both the standard deviation of the mean depth of the individual embossed elements in the form of dots, and the ratio between Standard deviation and Mean individual depth of a continuous embossed line extending along a contour of the absorbent sanitary napkin framing the embossed pattern comprising the individual embossed elements shows that the air-through-bonded nonwoven surface material has more distinct embossed elements that enhances the visibility of the embossing pattern when provided on the material.

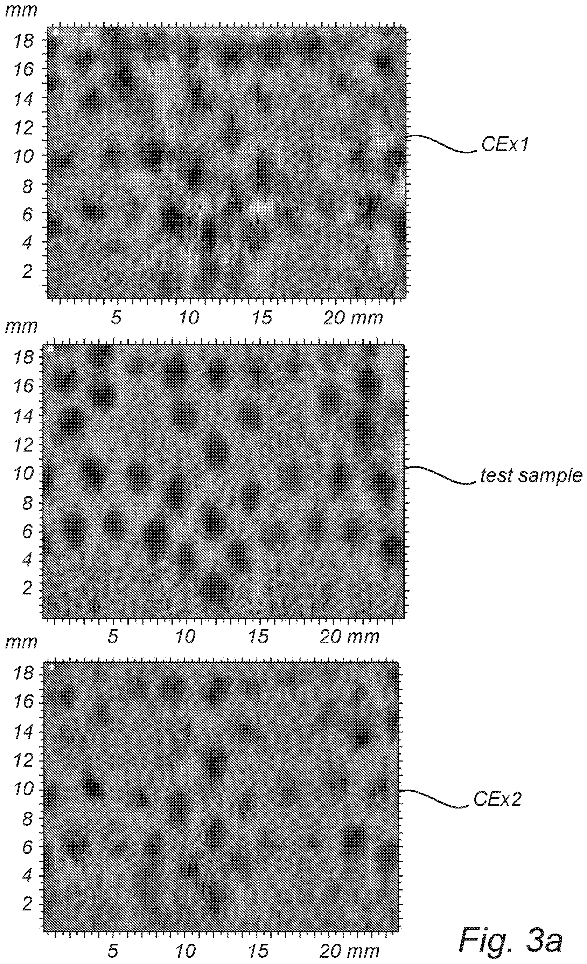

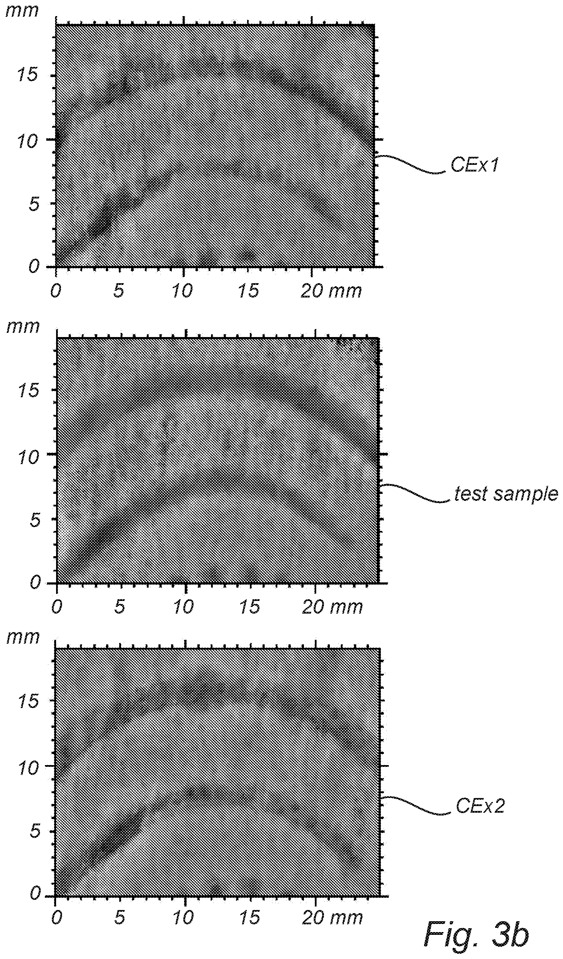

Also, the enlarged photos in figure 3a and 3b shows that the sanitary napkin with the test sample (air-through-bonded nonwoven) as surface material has more distinct embossed elements that enhances the visibility of the embossing pattern than the Comparative example 1 (Cex 1) and the Comparative example 2 (Cex 2).

### Friction measurement

Friction occurring between a nonwoven material and the skin of the user is different in the presence of liquid/moisture than when no liquid/moisture is present. Even a very small amount of moisture present originating from perspiration, sweat or other body fluids has an impact on the friction forces occurred between the nonwoven material and the skin of the user. It has therefore been discovered that it is really important to carefully choose the nonwoven characteristics, so that the nonwoven is able to minimize the mechanical discomfort during the overall use of the product.

The method used for the friction measurement was the Stick and slip measurement method which is described in detail in WO 2016/114693. The friction measurement has been performed according to the description in WO 2016/114693.

The method measures the static friction, sns value (stick and slip value) in gram force, gmf, between a material and the human skin. The method means that repeatedly runs are made using the same material strip. First the sns value for the dry state (dry material and skin) is measured followed by wet state at different liquid levels (from completely wetted material, to moist and to almost dry) until the sns value is back to the skin-material interaction level measured in the first dry run, which mean that the material is dry again. The method is thus called a repeated stick and slip method or sns dry-wet-dry. The stick and slip value is defined as the point on the force curve (gmf) where the material starts gliding over the arm. The sns values from all single force curves are then put together in a new graph, sns values as a function of number of runs.

Three different nonwoven materials were tested and compared in terms of dry friction and wet friction. The test material is an air-through-bonded nonwoven according to the present disclosure comprising bicomponent fibers of core-sheath type with a polyester core and a polyethylene sheath. The first Comparative Example is a spunbond nonwoven with polypropylene fibers and the second Comparative Example is spunbond nonwoven with polypropylene fibers. Table 1 above provides specifications of the materials tested.

In table 4 below shows result of the mean friction plateau values measured in gmf. By gmf is meant gram-force and one gram-force is 9.80665 mN and the result shows that the test sample, the air-through-bonded nonwoven surface material has a lower mean friction plateau value (gmf).

**Table 4**

| Material | Mean friction plateau value, (gmf) |
|---|---|
| Test sample | 300 |
| CEx 1 | 480 |
| CEx 2 | 420 |

Also in Figure 4 shows the friction curves for test sample, CEx 1 and CEx2 and the result shows that the test sample, the air-through-bonded nonwoven surface material, has a lower mean friction plateau value (gmf).

### Density measurement

The density is calculated by dividing the basis weight of the surface layer by its thickness. The thickness is determined by means of a measuring foot with affixed load of 0.5 k Pa. The measuring foot has an area of 50x50 mm². The thickness is read off at the digital thickness gauge/tester after 10 seconds when the measuring foot has touched the surface of the sample.

## Claims

1. An absorbent article (1) having longitudinal side edges (2,3) extending in a longitudinal direction (L) and transverse front and rear end edges (4,5), said absorbent article (1) comprising a fluid permeable surface layer (8) and a backsheet (9), said fluid permeable surface layer being an embossed fluid permeable surface layer (8) comprising an embossing pattern (11) covering from 3% to 20% of the total surface area of a wearer-facing portion of said surface layer (8), wherein said surface layer (8) is an air-through-bonded fibrous nonwoven surface layer (8) comprising synthetic fibers and having a basis weight of from 14 to 30 g/m², **characterized in that**
said surface layer (8) has a density of from 20 to 90 kg/m³, or 20 to 60 kg/m³ or 20 to 40 kg/m³, the density being calculated by dividing the basis weight of the surface layer (8) by its thickness measured at a pressure of 0.5 kPa.

2. The absorbent article (1) according to claim 1, wherein said embossing pattern (11) comprises embossed discontinuous dots having a minimum diameter of 0.3 mm, 0.6 mm or 0.9 mm and/or embossed continuous lines having a minimum width of 0.3 mm, 0.6 mm or 0.9 mm.

3. The absorbent article (1) according to claim 1 or 2, wherein said embossing pattern has a minimum depth of 0.3 mm or 0.5 mm, the depth being measured by the method ISO25178.

4. The absorbent article (1) according to any one of the preceding claims, wherein said synthetic fibers in said embossing pattern are permanently deformed but not consolidated.

5. The absorbent article (1) according to any one of the preceding claims, wherein the nonwoven surface layer (8) comprises bicomponent fibers.

6. The absorbent article (1) according to preceding claim, wherein said bi-component fibers are sheath-core bicomponent fibers, wherein said said sheath is a polyethylene sheath.

7. The absorbent article (1) according to claim 5 or 6, wherein said bi-component fibers are sheath-core bicomponent fibers, wherein said core is a polyester core and said sheath is a polyethylene sheath.

8. The absorbent article (1) according to any one of the preceding claims, wherein said embossing pattern covering from 5% to 16% of the total surface area of said wearer-facing portion of said surface layer (8).

9. The absorbent article (1) according to any one of the preceding claims, wherein said fibers of said air-through-bonded nonwoven have a coarseness of from 1.8 to 10 dtex, or 2 to 7 dtex.

10. The absorbent article (1) according to any one of the preceding claims, wherein said surface layer (8) is free from lotions and/or lubricating agents.

11. The absorbent article (1) according to any one of the preceding claims, wherein said absorbent article (1) comprises an intermediate layer (10) located between said surface layer (8) and said backsheet (9).

12. The absorbent article (1) according to claim 11, wherein said intermediate layer (10) covers from 70% to 100% of said surface layer (8).

13. The absorbent article (1) according to claims 11 or 12, wherein said surface layer (8) and said intermediate layer (10) are adhesively attached to each other.

14. The absorbent article (1) according to any one of the preceding claims, wherein said absorbent article (1) is a sanitary napkin.

## Patentansprüche

1. Saugfähiger Artikel (1), der in einer Längsrichtung (L) verlaufende Längsseitenränder (2, 3) und quer verlaufende vordere und hintere Endränder (4, 5) aufweist, wobei der saugfähige Artikel (1) eine flüssigkeitsdurchlässige Oberflächenlage (8) und eine Rückseitenlage (9) umfasst wobei die flüssigkeitsdurchlässige Oberflächenlage eine geprägte flüssigkeitsdurchlässige Oberflächenlage (8) ist, die ein Prägemuster (11) umfasst, das 3% bis 20% der Gesamtoberfläche eines dem Träger zugewandten Teils der Oberflächenlage (8) bedeckt, wobei die Oberflächenlage (8) eine luftdurchlässig verfestigte Faservlies-Oberflächenlage (8) ist, die synthetische Fasern umfasst und ein Basisgewicht von 14 bis 30 g/m² aufweist, **dadurch gekennzeichnet, dass**
die Oberflächenlage (8) eine Dichte von 20 bis 90 kg/m³, oder von 20 bis 60 kg/m³ oder von 20 bis 40 kg/m³ aufweist, wobei die Dichte durch Teilen des Basisgewichts der Oberflächenlage (8) durch ihre bei einem Druck von 0,5 kPa gemessene Dicke berechnet wird.

2. Saugfähiger Artikel (1) nach Anspruch 1, wobei das Prägemuster (11) geprägte unterbrochene Punkte mit einem Mindestdurchmesser von 0,3 mm, 0,6 mm oder 0,9 mm und/oder geprägte durchgehende Linien mit einer Mindestbreite von 0,3 mm, 0,6 mm oder 0,9 mm umfasst.

3. Saugfähiger Artikel (1) nach Anspruch 1 oder 2, wobei das Prägemuster eine Mindesttiefe von 0,3 mm oder 0,5 mm aufweist, wobei die Tiefe nach dem Verfahren gemäß ISO25178 gemessen wird.

4. Saugfähiger Artikel (1) nach einem der vorstehenden Ansprüche, wobei die synthetischen Fasern in dem Prägemuster dauerhaft verformt, jedoch nicht konsolidiert sind.

5. Saugfähiger Artikel (1) nach einem der vorstehenden Ansprüche, wobei die Faservlies-Oberflächenlage (8) Zweikomponentenfasern umfasst.

6. Saugfähiger Artikel (1) nach dem vorstehenden Anspruch, wobei es sich bei den Zweikomponentenfasern um Kern-Mantel-Zweikomponentenfasern handelt, wobei es sich bei dem Mantel um einen Polyethylen-Mantel handelt.

7. Saugfähiger Artikel (1) nach Anspruch 5 oder 6, wobei es sich bei den Zweikomponentenfasern um Kern-Mantel-Zweikomponentenfasern handelt, wobei es sich bei dem Kern um einen Polyester-Kern handelt und bei dem Mantel um einen Polyethylen-Mantel handelt.

8. Saugfähiger Artikel (1) nach einem der vorstehenden Ansprüche, wobei das Prägemuster von 5% bis 16% der Gesamtoberfläche des dem Träger zugewandten Teils der Oberflächenlage (8) bedeckt.

9. Saugfähiger Artikel (1) nach einem der vorstehenden Ansprüche, wobei die Fasern des luftdurchlässig verfestigten Vlieses eine Faserfeinheit von 1,8 bis 10 dtex oder 2 bis 7 dtex aufweisen.

10. Saugfähiger Artikel (1) nach einem der vorstehenden Ansprüche, wobei die Oberflächenlage (8) frei von Lotionen und/oder Schmiermitteln ist.

11. Saugfähiger Artikel (1) nach einem der vorstehenden Ansprüche, wobei der saugfähige Artikel (1) eine Zwischenlage (10) umfasst, die zwischen der Oberflächenlage (8) und der Rückseitenlage (9) angeordnet ist.

12. Saugfähiger Artikel (1) nach Anspruch 11, wobei die Zwischenlage (10) von 70% bis 100% der Oberflächenlage (8) bedeckt.

13. Saugfähiger Artikel (1) nach den Ansprüchen 11 oder 12, wobei die Oberflächenlage (8) und die Zwischenlage (10) haftend aneinander befestigt sind.

14. Saugfähiger Artikel (1) nach einem der vorstehenden Ansprüche, wobei es sich bei dem saugfähigen Artikel (1) um eine Damenbinde handelt.

## Revendications

1. Article absorbant (1) présentant des bords latéraux longitudinaux (2,3) s'étendant dans une direction longitudinale (L) et des bords d'extrémité avant et arrière transversaux (4,5), ledit article absorbant (1) comprenant une couche de surface (8) perméable aux fluides et une feuille arrière (9), ladite couche de surface perméable aux fluides étant une couche de surface (8) perméable aux fluides gaufrée comprenant un motif de gaufrage (11) couvrant de 3 % à 20 % de la surface totale d'une partie de ladite couche de surface (8), orientée vers le porteur, dans lequel ladite couche de surface (8) est une couche de surface (8) non tissée fibreuse liée par air traversant comprenant des fibres synthétiques et présentant un grammage de 14 à 30 g/m², **caractérisé en ce que**
ladite couche de surface (8) présente une densité de 20 à 90 kg/m³, ou de 20 à 60 kg/m³ ou de 20 à 40 kg/m³, la densité étant calculée en divisant le grammage de la couche de surface (8) par son épaisseur mesurée à une pression de 0,5 kPa.

2. Article absorbant (1) selon la revendication 1, dans lequel ledit motif de gaufrage (11) comprend des points discontinus gaufrés présentant un diamètre minimal de 0,3 mm, 0,6 mm ou 0,9 mm et/ou des lignes continues gaufrées présentant une largeur minimale de 0,3 mm, 0,6 mm ou 0,9 mm.

3. Article absorbant (1) selon la revendication 1 ou 2, dans lequel ledit motif de gaufrage présente une profondeur minimale de 0,3 mm ou 0,5 mm, la profondeur étant mesurée par la méthode ISO25178.

4. Article absorbant (1) selon l'une quelconque des revendications précédentes, dans lequel lesdites fibres synthétiques dans ledit motif de gaufrage sont déformées de façon permanente mais non consolidées.

5. Article absorbant (1) selon l'une quelconque des revendications précédentes, dans lequel la couche de surface (8) non tissée comprend des fibres bi-composant.

6. Article absorbant (1) selon la revendication précédente, dans lequel lesdites fibres bi-composant sont des fibres bi-composant gaine-âme, dans lequel ladite gaine est une gaine en polyéthylène.

7. Article absorbant (1) selon la revendication 5 ou 6, dans lequel lesdites fibres bi-composant sont des fibres bi-composant gaine-âme, dans lequel ladite âme est une âme en polyester et ladite gaine est une gaine en polyéthylène.

8. Article absorbant (1) selon l'une quelconque des revendications précédentes, dans lequel ledit motif de gaufrage couvre de 5 % à 16 % de la surface totale de ladite partie de ladite couche de surface (8), orientée vers l'utilisateur.

9. Article absorbant (1) selon l'une quelconque des revendications précédentes, dans lequel lesdites fibres dudit non-tissé lié par air traversant présentent un titre de 1,8 à 10 dtex, ou de 2 à 7 dtex.

10. Article absorbant (1) selon l'une quelconque des revendications précédentes, dans lequel ladite couche de surface (8) est exempte de lotions et/ou d'agents lubrifiants.

11. Article absorbant (1) selon l'une quelconque des revendications précédentes, dans lequel ledit article absorbant (1) comprend une couche intermédiaire (10) située entre ladite couche de surface (8) et ladite feuille arrière (9).

12. Article absorbant (1) selon la revendication 11, dans lequel ladite couche intermédiaire (10) couvre de 70% à 100% de ladite couche de surface (8).

13. Article absorbant (1) selon les revendications 11 ou 12, dans lequel ladite couche de surface (8) et ladite couche intermédiaire (10) sont fixées l'une à l'autre par adhésif.

14. Article absorbant (1) selon l'une quelconque des revendications précédentes, dans lequel ledit article absorbant (1) est une serviette hygiénique.
